Europäisches Patentamt

European Patent Office

Office européen des brevets

(11) Publication number: **0 084 333**
**B1**

(12) **EUROPEAN PATENT SPECIFICATION**

(45) Date of publication of patent specification: **11.06.86**

(21) Application number: **83100111.0**

(22) Date of filing: **08.01.83**

(51) Int. Cl.⁴: **C 12 P 19/26,** A 61 K 31/73 // C08B37/00

(54) Polysaccharide substance, process for the production of same, pharmaceutical compositions containing the same and their use as medicaments.

(30) Priority: **14.01.82 JP 3410/82**

(43) Date of publication of application:
**27.07.83 Bulletin 83/30**

(45) Publication of the grant of the patent:
**11.06.86 Bulletin 86/24**

(84) Designated Contracting States:
**AT BE CH DE FR GB IT LI NL SE**

(56) References cited:
**CHEMICAL ABSTRACTS, vol. 90, no. 7, 12th February 1979, page 297, no. 51201g, Columbus Ohio (USA); R.L.MacLEOD et al.: "Translocation of lipopolysaccharine in the cell wall of a gram-negative marine bacterium ".**

**CHEMICAL ABSTRACTS, vol. 89, no. 25, 18th December 1978, page 281, no. 211626j, Columbus Ohio (USA);J.M.DiRIENZO et al.: "Composition of the fractions separated by polyacrylamide gel electrophoresis of the lipopolysaccharide of a marine bacterium"**

(73) Proprietor: **MICROBIAL CHEMISTRY RESEARCH FOUNDATION**
**14-23, Kamiosaki 3 Chome Shinagawa-ku**
**Tokyo 141 (JP)**

(72) Inventor: **Umezawa, Hamao, Prof.**
**4-chome-23, Toyotama-kita Nerima-ku**
**Tokyo (JP)**
Inventor: **Okami, Yoshiro**
**4-18-14, Denenchofu Ohta-ku**
**Tokyo (JP)**
Inventor: **Takeuchi, Tomio**
**5-1-11, Higashi-Gotanda Shinagawa-ku**
**Tokyo (JP)**
Inventor: **Kurasawa, Shogo**
**5-39-2, Sendagi Bunkyo-ku**
**Tokyo (JP)**
Inventor: **Yamada, Kazuhiko**
**3-22-22, Fujigaoka**
**Fujisawa-city Kanagawa Prefecture (JP)**
Inventor: **Suzuki, Katsumi**
**3-1631, Ikebukuro Toshima-ku**
**Tokyo (JP)**
Inventor: **Shiio, Tsuyoshi**
**1495-5, Yamazaki**
**Kamakura-city Kanagawa Prefecture (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 0 084 333

(58) References cited:
CHEMICAL ABSTRACTS, vol. 89, no. 25, 18th December 1978, page 281, no. 211625h, Columbus Ohio (USA);J.M.DiRIENZO et al.: "Heterogeneity and distribution of lipopolysaccharide in the cell wall of a gram-negative marine bacterium"

CHEMICAL ABSTRACTS, vol. 86, no. 17, 25th April 1977, page 229, no. 117305f, Columbus Ohio (USA); J.D.NELSON Jr. et al.: "Distribution of lipopolysaccharide and the detection of a new subfraction in the cell envelope of a marine pseudomonad"

CHEMICAL ABSTRACTS, vol. 91, no. 7, 13th August 1979, page 323, no. 52415n, Columbus Ohio (USA); K.Y. CAHN et al.: "Requirement of sodium by marine heterotrophic bacteria".

CHEMICAL ABSTRACTS, vol. 80, no. 17, 29th April 1974, page 178, no. 93051g, Columbus Ohio (USA); L. BAUMANN et al.: "Regulation of aspartokinase activity in nonfermentative marine eubacteria"

CHEMICAL ABSTRACTS, vol. 86, no. 1, 3rd January 1977, page 192, no. 2073a, Columbus Ohio (USA); M.J.GAUTHIER et al.: "Antibacterial activity of marine violet-pigmented alteromonas with special reference to the production of brominated compounds"

CHEMICAL ABSTRACTS, vol. 88, no. 5, 30th January 1978, page 230, no. 34276b, Columbus Ohio (USA); M.J.GAUTHIER: "Alteromonas citrea, a new gram-negative, yellow-pigmented species from seawater"

CHEMICAL ABSTRACTS, vol. 96, no. 21, 24th May 1982, page 389, no. 177631q, Columbus Ohio (USA); R.A.LADDAGA et al.: "Effects of wash treatments on the ultrastructure and lysozyme penetrability of the outer membrane of various marine and two terrestrial gram-negative bacteria"

JOURNAL OF BACTERIOLOGY, vol. 110, no. 1, April 1972, pages 402-429, (USA); L.BAUMANN et al.: "Taxonomy of aerobic marine eubacteria.

(72) Inventor: Ishizuka, Masaaki
3-17, Denenchofu-Honcho Ohta-ku
Tokyo (JP)
Inventor: Ohnuki, Takashi
1155-2, Nakamaruko Nakahara-ku
Kawasaki-city Kanagawa Prefecture (JP)

(74) Representative: Reuter, Johann-Heinrich, Dr. et al
HOECHST AKTIENGESELLSCHAFT Zentrale
Patentabteilung Postfach 80 03 20
D-6230 Frankfurt/Main 80 (DE)

# 0 084 333

**Description**

This invention relates to the polysaccharide substance MP-67, a process for the production of the same, and their use as medicaments.

A number of polysaccharides have heretofore been discovered, and it is known that they have a wide variety of uses. The present invention is based on the discovery that a specific microorganism produces the novel polysaccharide substance MP-67, and this substance has anti-tumor activity and immunopotentiating activity.

The polysaccharide substance MP-67 of this invention shows the following analysis and properties:

a. A carbon to nitrogen ratio, determined by elemental analysis, of 20:1, although accurate analysis is difficult because said substance is hygroscopic;

b. An average molecular weight, determined by gel filtration using Sepharose CL-4B®, of $2 \times 10^5$ to $4 \times 10^5$;

c. Insoluble in organic solvents and soluble in water;

d. Positive in a color development test by the phenol-sulfuric acid method;

e. The sugar composition determined by gas chromatography: $53 \pm 5\%$ rhamnose, $21 \pm 2\%$ glucose, $7.0 \pm 0.7\%$ mannose and $6.0 \pm 0.6\%$ galactose;

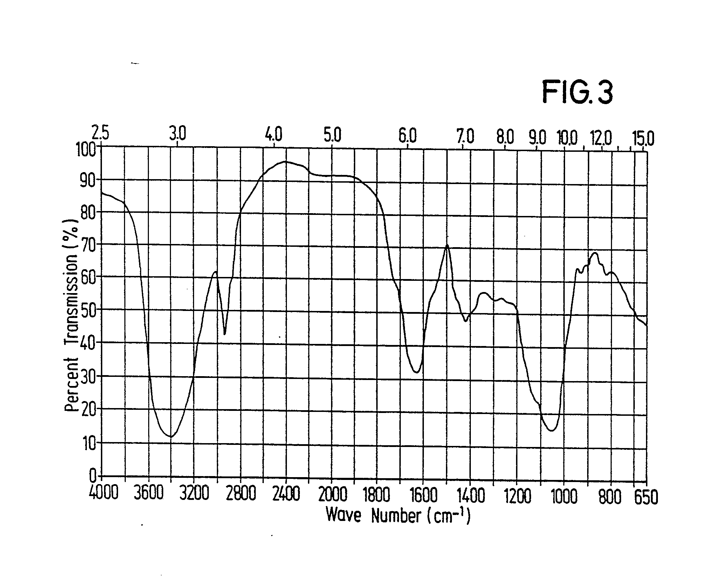

f. An infrared absorption spectrum as shown in Fig. 3, and

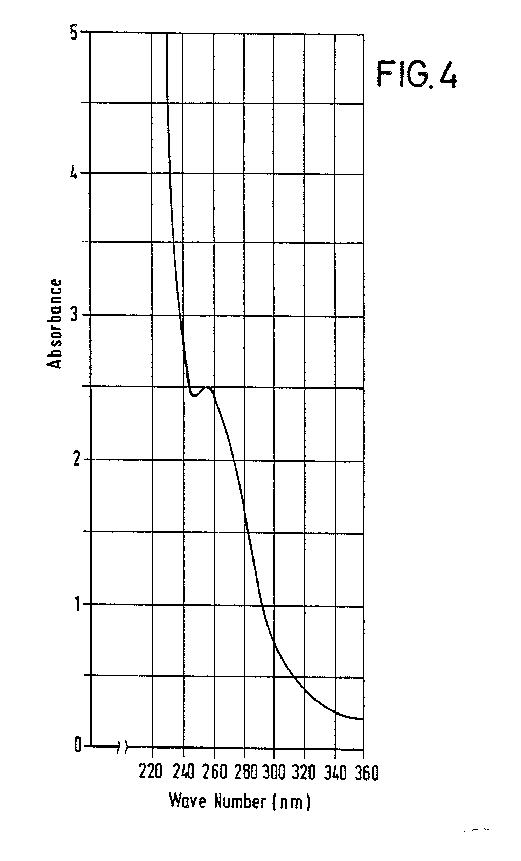

g. An ultraviolet absorption spectrum as shown in Fig. 4.

The polysaccharide substance MP-67 is present in the fermentation broth of a microorganism belonging to the genus Alteromonas and can be obtained by recovering therefrom. The polysaccharide substance MP-67 of this invention is produced by the cultivation of the microorganism *Alteromonas vaga* MP-67 (FERM P-6281) or one of its mutants capable of producing said polysaccharide in a medium consisting of carbon sources, nitrogen sources, inorganic ions, and if necessary, organic nutrients such as vitamins and amino acids, dissolved in seawater (including artificial seawater), and the polysaccharide substance MP-67 produced and accumulated in the fermentation broth is separated and recovered.

Any carbon sources which are usually used for the cultivation of microorganisms can be used in the present invention. Preferred examples of such carbon sources are carbohydrates such as glucose, sucrose, starch and dextrin. Also, nitrogen sources which are usually used for the cultivation of microorganisms can be used in the present invention. Examples of such nitrogen sources include peptone, yeast extract, meat extract, corn steep liquor, soybean powder, casein and ammonium ions.

Cultivation is performed preferably under aerated conditions with stirring. The cultivation temperature can be appropriately selected within a range which permits the MP-67-producing microorganism to grow and produce the polysaccharide substance MP-67, and it is usually preferred to be from 10°C to 35°C and more preferably from 24°C to 30°C. The cultivation is performed until a sufficient amount of the desired polysaccharide substance MP-67 is accumulated in the fermentation broth. Usually, the cultivation time is from 15 hours to 100 hours.

The microorganism capable of producing the polysaccharide substance MP-67 of the present invention is *Alteromonas vaga* MP-67 (FERM P-6281).

This strain is collected and isolated from marine environment and has the following microbiological characteristics:

a. Morphological Characteristics
1. Form and size of the cell: Rod and 0.3 to 0.5 × 0.7 to 0.9 μm
2. Pleomorphism: Non-pleomorphic
3. Motility/flagellation: Motile/polar flagella
4. Sporogenicity: Non-sporulating
5. Gram's stain: Negative
6. Acid-fastness: Negative

b. Culture Characteristics on Various Media
1. Bouillon agar plate culture: No growth
1'. Marine agar 2216 medium plate culture: Medium growth, circular, flat to semilenticular, entire margin, smooth, glistening, translucent, butyraceous, and pale yellow to light yellow.
2. Bouillon agar slant culture: No growth
2'. Marine agar 2216 medium slant culture: Moderate growth, membranous, thread-like, glistening, and pale yellow to light yellow
3. Bouillon liquid culture: No growth
3'. Marine 2216 medium liquid culture: Slightly turbid, no membrane formation on the surface, and powder precipitation
4. Bouillon gelatin stab culture: No changes
4'. Marine 2116 medium gelatin stab culture: Liquefaction
5. Litmus milk: No changes.

3

# 0 084 333

c. Physiological Characteristics

1. Reduction of nitrates: Negative
2. Denitrification: Negative
3. MR test: Negative
4. VP test: Negative
5. Production of indole: Negative
6. Production of hydrogen sulfide: Negative
7. Hydrolysis of starch: Negative
8. Utilization of citric acid: Positive on a Koser medium, and positive on a Christensen medium.
9. Utilization of inorganic nitrogen source: Utilizes nitrates and ammonium salts.
10. Production of pigment: No formation of water-soluble pigment
11. Urease: Negative
12. Oxidase: Negative
13. Catalase: Positive
14. Growth ranges: Temperature: Good growth at 14°C to 37°C; optimum temperature: 26°C to 34°C; and no growth at 40°C. pH: 6 to 9.
15. Oxygen demand: Aerobic
16. O—F test (according to the Hugh & Leifson method): No acid is formed.
17. Production of acids and gases from Saccharides:

| Saccharides | Production of Acid | Production of Gas |
|---|---|---|
| L-Arabinose | − | − |
| D-Xylose | + | − |
| D-Glucose | +* | − |
| D-Mannose | +* | − |
| D-Fructose | +* | − |
| D-Galactose | − | − |
| Maltose | +* | − |
| Sucrose | − | − |
| Lactose | − | − |
| Trehalose | − | − |
| D-Sorbitol | − | − |
| D-Mannitol | + | − |
| Inositol | − | − |
| Glycerin | + | − |
| Starch | − | − |
| Raffinose | − | − |
| Cellobiose | ± | − |
| D-Ribose | − | − |
| Rhamnose | − | − |

* Slight production

4

18. Arginine dihydrolase reaction (according to the method of Stanier et al.): Negative
19. Decomposition of casein: Negative
20. Accumulation of poly-hydroxybutyric acid: Negative
21. Nutrient requirement: No growth occurs unless seawater is added.
22. Utilization of the following compounds (Stanier medium + Marine Salts)

| D-Glucose | + | Butyric acid | − |
| L-Arabinose | ± | m-Benzoic acid | − |
| D-Xylose | + | p-Benzoic acid | − |
| D-Mannose | + | meso-Tartaric acid | − |
| D-Galactose | − | Levulinic acid | − |
| Saccharose | − | Citraconic acid | − |
| Lactose | − | Gluconic acid | + |
| Cellobiose | ± | Malonic acid | − |
| Salicin | − | L-Alanine | − |
| Erythritol | ± | -Alanine | − |
| D-Mannitol | + | L-Valine | − |
| Glycerine | + | L-Ornithine | + |
| D-Fructose | + | L-Lysine | + |
| Inositol | + | L-Tyrosine | − |
| Trehalose | ± | DL-Arginine | ± |
| Acetic acid | + | Betaine | ± |
| Succinic acid | + | Ethanol | − |
| Citric acid | + | DL-Hydroxybutyrate | + |
| Lactic acid | + | Fumaric acid | + |
| Propionic acid | − | -Ketoglutaric acid | + |
| | | L-Histidine | + |

23. GC content of DNA: 46.2%.

In view of the above microbiological characteristics, Bergey's Manual of Determinative Bacteriology, 8th Ed. has been referred to for the identification of the strain used in the present invention. This strain does not agree with the genus Pseudomonas having a DNA—GC content of 58 to 70%. Vibrio, Photobacterium, Beneckea and Lucibacterium species are inconsistent with the strain because these species are facultative anaerobes and the other characteristics of them are also different from those of the strain. Acinetobacter and Moraxella species, non-motile bacteria, differ from the strain. Bdelloxibrio species, obligatory parasites, do not conform to the strain. Halobacterium species, having a DNA—GC content of 57 to 68%, do not agree with the strain. The strain of the invention thus differs from the genera described in said manual in terms of important characteristics and does not conform to any of the genera.

Search through taxonomic systems other than revealed in said manual has shown the strain of the invention to be in good agreement with the genus *Alteromonas* (Gram-negative, rod, non-fermentative, polar flagellate, DNA—GC content of 40 to 50%, marine bacteria, aerobic) proposed by Baumann et al. (Taxonomy of aerobic marine eubacteria, J. Bacteriol. *110*, 402—429, 1972). This strain has therefore been identified as a bacterium belonging to the genus Alteromonas. Among the known species of this genus,

**0 084 333**

*Alteromonas vaga* (Baumann et al., 1972) is in accordance with the strain of the present invention possessing the following characteristics: a DNA—GC content of 46.2%, negativeness of oxidase, no growth at 40°C, and no decomposition of starch. The present strain has been deposited in the Fermentation Research Institute, Agency of Industrial Science and Technology, Ministry of International Trade and Industry of Japan under the acccession number of FERM P-6281. Any mutants obtained either by artificially such as by irradiation with ultraviolet rays or by X-rays, treatment with chemicals, or by spontaneous mutation can be used in the present invention as long as they have the ability to produce the polysaccharide substance MP-67 of the present invention. The polysaccharide substance MP-67 of this invention can be removed from the fermentation broth by known methods. For example, the fermentation broth is subjected to centrifugal separation at 7,500 rpm for fifteen minutes to provide a supernatant liquid. A lower alkanol, preferably ethanol is added to the supernatant liquid, and the resulting mixture is allowed to stand in an ice chamber to cause precipitation. The precipitate is isolated, and dried under reduced pressure to obtain a crude form of the polysaccharide substance MP-67.

The crude polysaccharide substance MP-67 thus obtained is then further purified. For this purpose, various methods can be used. For example, chloroform and n-butanol are added to an aqueous solution of the crude substance, and the resulting mixture is stirred and then subjected to centrifugal separation to obtain a supernatant liquid. The same procedure as above is applied repeatedly once to several times to the supernatant liquid. To the supernatant liquid thus obtained is added ethanol to precipitate a purified form of the polysaccharide substance MP-67. A 0.05 N—NaCl solution of this purified substance is passed through a Sepharose CL-4B® column and eluted with 0.05 N—NaCl at a suitable flow rate. The eluate is fractionated in a predetermined amount, and MP-67-containing fractions are collected and dialyzed to remove NaCl therefrom. Then, ethanol is added to cause precipitation whereby a purified MP-67 fraction can be obtained. The physical and chemical properties of the polysaccharide substance MP-67 of this invention are described below. 0.5 ml of 72% sulfuric acid was added to 10 mg of the polysaccharide substance MP-67, and the mixture was allowed to stand for two hours at room temperature. To the mixture was added 7 ml of water, and the resulting mixture was placed in a sealed tube. The tube was heated in an oil bath at 105°C for two hours. The reaction mixture was neutralized with barium hydroxide, and the resulting barium sulfate was removed by filtration. The filtrate was dried under reduced pressure, and trimethylsilylated with a commercially available trimethylsilylating agent. Gas chromatographic analysis with reference to a standard sample shows that the polysaccharide substance MP-67 of this invention has the composition given in Table 1.

TABLE 1

| | |
|---|---|
| Rhamnose | 53 ± 5% |
| Glucose | 21 ± 2% |
| Mannose | 7.0 ± 0.7% |
| Galactose | 6.0 ± 0.6% |

Similar to other polysaccharides, the polysaccharide substance MP-67 of this invention is hygroscopic, and thus difficult to analyze accurately. One example of its elemental analysis is C: 39.87%; H: 6.89%; and N: 1.98%. The carbon/nitrogen ratio of the polysaccharide substance MP-67 of this invention is C: N = 20:1.

The average molecular weight determined by a gel filtration method using Sepharose CL-4B® is $2 \times 10^5$ to $4 \times 10^5$. The infrared absorption spectrum employing the KBr tablet is shown in Fig. 3. The ultraviolet absorption spectrum for an aqueous solution (1 mg/ml) is shown in Fig. 4. The polysaccharide substance MP-67 of the present invention is white. This substance is insoluble in organic solvents, but soluble in water. The substance is positive in a color development test by the phenol-sulfuric acid method. The components of the polysaccharide substance MP-67 of this invention contain an unidentified substance. To identify this substance, further investigation was carried out in the following way: 1 ml of $1N—H_2SO_4$ was added to about 5 mg of Sample III to be described later. The mixture was placed in a sealed tube, and heated in an oil bath at 100°C for five hours for hydrolysis. After cooling, the reaction mixture was neutralized with a saturated solution of barium hydroxide, and centrifuged at 3000 rpm for fifteen minutes to remove the precipitate formed. The supernatant was lyophilized and dissolved in 0.5 ml of water. The solution was subjected to thinlayer chromatography on a plate (Kieselgel 60 F, a product of Merck), using a 4:4:2 mixture of isopropanol, acetone and 0.1 M lactic acid as a developing agent. The plate had been dipped in a 0.5 M $NaH_2PO_4$ solution, air-dried, and heated at 105°C for one hour. The thinlayer chromatography gave the results shown in Table 2. The sugars were detected by spraying the plate with an anisaldehyde-sulfuric acid solution, followed by heating the sprayed plate at 105°C.

6

# 0 084 333

TABLE 2

| Standard sample | | | Product of the invention | |
|---|---|---|---|---|
| Sugar | Rf | Color | Rf | Color |
| Rhamnose | 0.60 | Yellow | 0.60 | Yellow |
| Mannose | 0.36 | Brown | 0.36 | Brown |
| Glucose | 0.30 | Blue | 0.30 | Blue |
| | | | 0.27 | Brown |
| Galactose | 0.23 | Bluish green | 0.23 | Bluish green |

The substance having an Rf of 0.27 was compared with arabinose, sorbose, glucoheptose, talose, altrose, tagatose, fructose, and ribose. However, this substance did not conform to any of these sugars.

The polysaccharide substance MP-67 of this invention has very strong immunopotentiating activity, and it is therefore useful for prevention and treatment of infectious diseases. In addition, this substance exhibits its anti-cancer effect mediated by immunological response of host. This invention is described in greater detail below with reference to the following examples.

## Example 1

*Alteromonas vaga* MP-67 strain (FERM P-6281) was inoculated in a 30-liter jar fermentor containing 15 liters of a medium (pH 7.2) of artificial seawater (Jamarin S, a product of Jamarin Laboratory) containing 0.8% glucose, 0.5% polypeptone and 0.1% yeast extract, and incubated at 27°C for 23 hours under stirring at 300 rpm and aeration at 1/3 vvm.

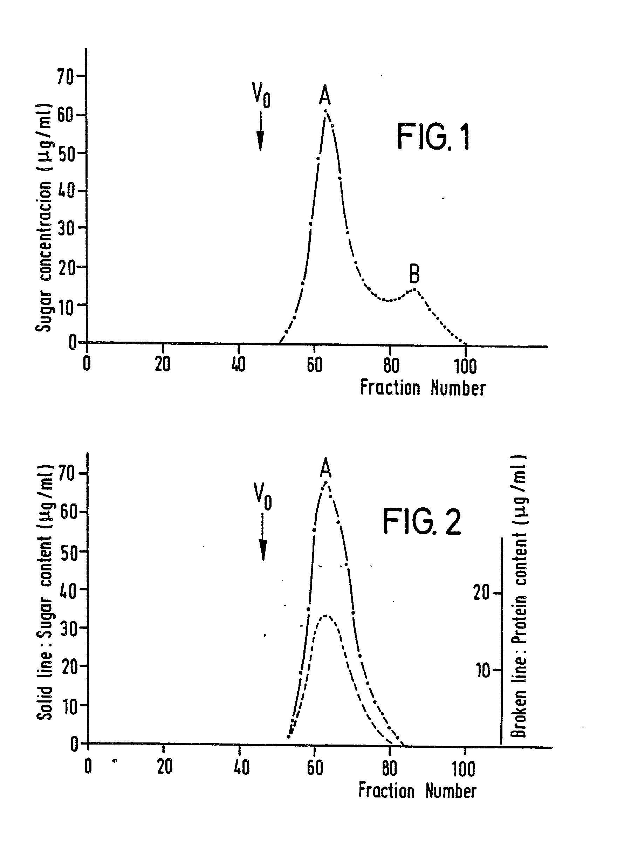

The fermentation broth was centrifuged at 7500 rpm for fifteen minutes to remove the bacterial cell and to obtain a supernatant. To the supernatant was added a 1.5-fold volume of ethanol. The mixture was allowed to stand overnight in an ice chamber to cause precipitation. The precipitate thus formed was collected, and dried under reduced pressure to obtain a crude form of the polysaccharide substance MP-67 (hereinafter referred to as Sample I) in a yield of 4.47 g. The crude polysaccharide substance MP-67 had a sugar content, determined by the phenolsulfuric acid method (standard sample:glucose), of 35.6% and a protein content, determined by the Lowry method (standard sample:bovine serum albumin), of 11.6%. 1 g of Sample I was dissolved in 200 ml of 0.05 M—NaCl, and each 10 ml, per chromatography, of this solution was passed through a Sepharose CL-6B® column (7.5 cm 0 × 60 cm, 0.05 M—Na—Cl), and eluted with 0.05 M—NaCl at a flow rate of 80 ml/hr. The eluate was fractionated into fractions of every 20 ml. Fig. 1 shows the respective gel filtration chromatogram. Fraction A (shown in Fig.) was collected.

Fraction A was concentrated and dialyzed. To 58 ml of the dialyzate was added ethanol in an amount of 2 times the amount of dialyzate to cause precipitation. The precipitate formed was recovered and dried under reduced pressure to obtain dry mass (hereinafter referred to as Sample II) in a yield of 500 mg. The same analyses as for Sample I showed that Sample II has a sugar content of 36.1% and a protein content of 8.6%. 320 mg of Sample II was dissolved in 32 ml of 0.05 N—NaCl, and the solution was purified by gel filtration in the same by way as described above except that 5 ml of the solution was used. The resulting purified sample solution was dialyzed three times against 5 liters of deionized water, and the dialyzate was desalted by passage through a Sephadex G-50® (5 cm 0 × 40 cm) column. The resulting eluate was then concentrated into 10 ml. The concentrate was dialyzed against deionized water, and ethanol in an amount of two times the amount of the dialyzate was added to the dialyzate to cause precipitation. The precipitate formed was dried under reduced pressure to obtain 140 mg of a pure polysaccharide product (hereinafter referred to as Sample III) as white powder. This product had a sugar content of 38.0% and a protein content of 8%. A chromatogram of Sample III is shown in Fig. 2. Sample III had the aforementioned physical and chemical properties.

## Example 2

A 3-liter flask was charged with .1 liter of a medium having the same composition as in Example 1 except that the medium had a glucose content of 0.6%. The same strain as shown in Example 1 was inoculated into the medium, and incubated at 27°C for three days under reciprocating shake. Then, the same procedure as used in Example 1 was employed to obtain 310 mg of the crude-form polysaccharide substance MP-67. This substance had a sugar content of 37% and a protein content of 13.3%.

7

# 0 084 333

Example 3

a. Antitumor test in Sarcoma-180 ascites tumor

To ICR mice (five weeks old, female) were intraperitoneally transplanted $1 \times 10^6$ Sarcoma-180 tumor cells. For 10 days from the day subsequent to the transplantation, a predetermined dose of sample were intraperitoneally administered. The average number of survival days and life-prolonged effect were determined. The results are shown in Table 3.

TABLE 3

| Sample | Dose (mg/kg × days) | Number of mice | Average survival days (days) | Increase of life span (%) |
|---|---|---|---|---|
| Control I | | 8 | 9.6 ± 1.8 | 100 |
| | 50 × 10 | 8 | 37.8 ± 17.0 | 394 |
| Control II | | 7 | 11.6 ± 3.2 | 100 |
| | 5 × 10 | 7 | 21.1 ± 9.5 | 181.9 |
| | 10 × 10 | 7 | 16.9 ± 8.5 | 130.2 |
| Control III | | 7 | 11.6 ± 3.2 | 100 |
| | 5 × 10 | 7 | 13.1 ± 3.5 | 112.9 |
| | 10 × 10 | 7 | 21.4 ± 8.0 | 184.5 |

b. Action on blastogenesis of spleen cells of mouse

Spleen cells were collected from $CDF_1$ mice (6 weeks old, female), and suspended in an RPMI 1640 medium containing 1% of fetal calf serum in a proportion of $1 \times 10^6$ cells per milliliter.

To 0.2 ml of the cell suspension was added 10 microliters of a sample solution, and the mixture was cultured at 37°C in the presence of 5%, $CO_2$. Fifty-four hours later, 0.1 uCi of $^3$H-thymidine was added, and cultivation was continued for an additional 18 hours. Then, the $^3$H-thymidine incorporation to the cultured spleen cells were determined by counting the radioactivity. The results are shown in Table 4.

TABLE 4

| Concentration of sample (µg/ml) | Count (cpm/culture) |
|---|---|
| 0 | 355 |
| 0.1 | 587 |
| 0.4 | 835 |
| 1.6 | 1108 |
| 6.2 | 2287 |
| 25.0 | 3402 |
| 100.0 | 2427 |

8

**Claims for the Contracting States: BE CH DE FR GB IT LI NL SE**

1. A polysaccharid substance having
a) a carbon to nitrogen ratio of 20:1;
b) an average molecular weight of $2 \times 10^5$ to $4 \times 10^5$;
c) the sugar composition $53 \pm 5\%$ rhamnose, $21 \pm 2\%$ glucose, $7.0 \pm 0.7\%$ mannose, $6.0 \pm 0.6\%$ galactose;
d) insolubility in organic solvents and solubility in water;
e) positiveness in a color development test by the phenol-sulfuric acid method;
f) an infrared absorption spectrum as shown in Fig. 3;
g) an ultraviolet absorption spectrum as shown in Fig. 4.

2. A process for producing a polysaccharid substance according to claim 1, which comprises cultivating the microorganism Alteromonas vaga MP-67, *FERM P-6281* or one of its mutants capable of producing the polysaccharide of claim 1, and then recovering the polysaccharid produced in nutrient media.

3. The process according to claim 2 wherein the microorganism is cultivated in a medium consisting of carbon sources, inorganic ions and optionally vitamins and amino acids dissolved in seawater, including artificial seawater, under aerated conditions at a temperature of from 10°C to 35°C and the polysaccharide produced and accumulated in the fermentation broth is separated and recovered.

4. The process according to claim 3 wherein the microorganism is cultivated at a temperature of from 24°C to 30°C.

5. The process according to claim 3 wherein the polysaccharide substance is recovered from the fermentation broth by subjecting the latter to centrifugal separation, adding an alkanol to the supernatant liquid, causing precipitation by cooling and isolating and purifying the precipitate by known methods.

6. The microorganism Alteromonas vaga MP-67, FERM P-6281.

7. A pharmaceutical composition comprising as active ingredient a polysaccharide substance as claimed in claim 1 in association with a pharmaceutically acceptable carrier.

8. A polysaccharide substance as claimed in claim 1 for use as a medicament.


**Claims for the Contracting State: AT**

1. A process for producing a polysaccharid substance having
a) a carbon to nitrogen ratio of 20:1;
b) an average molecular weight of $2 \times 10^5$ to $4 \times 10^5$;
c) the sugar composition $53 \pm 5\%$ rhamnose, $21 \pm 2\%$ glucose, $7.0 \pm 0.7\%$ mannose, $6.0 \pm 0.6\%$ galactose;
d) insolubility in organic solvents and solubility in water;
e) positiveness in a color development test by the phenol-sulfuric acid method;
f) an infrared absorption spectrum as shown in Fig. 3;
g) an ultraviolet absorption spectrum as shown in Fig. 4,
which comprises cultivating the microorganism Alteromonas vaga MP-67, *FERM P-6281* or one of its mutants capable of producing said polysaccharide, and then recovering the polysaccharid produced in nutrient media.

2. The process according to claim 1 wherein the microorganism is cultivated in a medium consisting of carbon sources, inorganic ions and optionally vitamins and amino acids dissolved in seawater, including artificial seawater, under aerated conditions at a temperature of from 10°C to 35°C and the polysaccharide produced and accumulated in the fermentation broth is separated and recovered.

3. The process according to claim 2 wherein the microorganism is cultivated at a temperature of from 24°C to 30°C.

4. The process according to claim 2 wherein the polysaccharide substance is recovered from the fermentation broth by subjecting the latter to centrifugal separation, adding an alkanol to the supernatant liquid, causing precipitation by cooling and isolating and purifying the precipitate by known methods.


**Patentansprüche für die Vertragsstaaten: BE CH DE FR GB IT LI NL SE**

1. Polysaccharidsubstanz mit
a) einem Kohlenstoff/Stickstoffverhältnis von 20:1,
b) einem Durchschnittsmolekulargewicht von $2 . 10^5$ bis $4 . 10^5$,
c) der Zuckerzusammensetzung $53 \pm 5\%$ Rhamnose, $21 \pm 2\%$ Glucose, $7,0 \pm 0,7\%$ Mannose und $6,0 \pm 0,6\%$ Galaktose,
d) Unlöslichkeit in organischen Lösungsmitteln und Löslichkeit in Wasser,
e) positivem Farbentwicklungstest nach der Phenolschwefelsäuremethode,
f) einem Infrarotabsorptionsspektrum wie in Abbildung 3 und
g) einem Ultraviolettabsorptionsspektrum wie in Abbildung 4.

**0 084 333**

2. Verfahren zur Herstellung einer Polysaccharidsubstanz nach Anspruch 1, dadurch gekennzeichnet, dass man den Mikroorganismus Alteromonas vaga MP-67, *FERM P-6281* oder eine seiner zur Bildung des Polysaccharids nach Anspruch 1 befähigten Mutanten kultiviert und dann das im Nährmedien gebildete Polysaccharid gewinnt.

3. Verfahren nach Anspruch 2, dadurch gekennzeichnet, dass man den Mikroorganismus in einem aus in Meerwasser bzw. künstlichem Meerwasser gelösten Kohlenstoffquellen, anorganischen Ionen und gegebenenfalls Vitaminen und Aminosäuren bestehenden Medium unter Luftzutritt bei einer Temperatur von 10°C bis 35°C kultiviert und das gebildete, in der Fermentationsbrühe angereicherte Polysaccharid abtrennt und gewinnt.

4. Verfahren nach Anspruch 3, dadurch gekennzeichnet, dass man den Mikroorganismus bei einer Temperatur von 24°C bis 30°C kultiviert.

5. Verfahren nach Anspruch 3, dadurch gekennzeichnet, dass man die Polysaccharidsubstanz dadurch aus der Fermentationsbrühe gewinnt, dass man diese einer Trennung durch Zentrifugieren unterwirft, der überstehenden Flüssigkeit ein Alkanol zusetzt, durch Abkühlen Ausfällung einleitet und den Niederschlag nach bekannten Methoden gewinnt und reinigt.

6. Der Mikroorganismus Alteromonas vaga MP-67, FERM P-6281.

7. Pharmazeutische Zusammensetzungen, dadurch gekennzeichnet, dass sie aus einer Polysaccharidsubstanz nach Anspruch 1 als Wirkstoff zusammen mit einem pharmazeutisch unbedenklichen Träger bestehen.

8. Polysaccharidsubstanz nach Anspruch 1 zur Verwendung als Arzneimittel.


**Patentansprüche für den Vertragsstaat: AT**

1. Verfahren zur Herstellung einer Polysaccharidsubstanz mit

a) einem Kohlenstoff/Stickstoffverhältnis von 20:1,

b) einem Durchschnittsmolekulargewicht von $2 \cdot 10^5$ bis $4 \cdot 10^5$,

c) der Zuckerzusammensetzung $53 \pm 5\%$ Rhamnose, $21 \pm 2\%$ Glucose, $7,0 \pm 0,7\%$ Mannose und $6,0 \pm 0,6\%$ Galaktose,

d) Unlöslichkeit in organischen Lösungsmitteln und Löslichkeit in Wasser,

e) positivem Farbentwicklungstest nach der Phenolschwefelsäuremethode,

f) einem Infrarotabsorptionsspektrum wie in Abbildung 3 und

g) einem Ultraviolettabsorptionsspektrum wie in Abbildung 4,

dadurch gekennzeichnet, dass man den Mikroorganismus Alteromonas vaga MP-67, *FERM P-6281* oder eine seiner zur Bildung dieses Polysaccharids befähigten Mutanten kultiviert und dann das im Nährmedien gebildete Polysaccharid gewinnt.

2. Verfahren nach Anspruch 1, dadurch gekennzeichnet, dass man den Mikroorganismus in einem aus in Meerwasser bzw. künstlichem Meerwasser gelösten Kohlenstoffquellen, anorganischen Ionen und gegebenenfalls Vitaminen und Aminosäuren bestehenden Medium unter Luftzutritt bei einer Temperatur von 10°C bis 35°C kultiviert und das gebildete, in der Fermentationsbrühe angereicherte Polysaccharid abtrennt und gewinnt.

3. Verfahren nach Anspruch 2, dadurch gekennzeichnet, dass man den Mikroorganismus bei einer Temperatur von 24°C bis 30°C kultiviert.

4. Verfahren nach Anspruch 2, dadurch gekennzeichnet, dass man die Polysaccharidsubstanz dadurch aus der Fermentationsbrühe gewinnt, dass man diese einer Trennung durch Zentrifugieren unterwirft, der überstehenden Flüssigkeit ein Alkanol zusetzt, durch Abkühlen Ausfällung einleitet und den Niederschlag nach bekannten Methoden gewinnt und reinigt.


**Revendications pour les Etats contractants: BE CH DE FR GB IT LI NL SE**

1. Substance à base de polysaccharide ayant

a) un rapport carbone/azote de 20:1,

b) une masse moléculaire moyenne de $2 \times 10^5$ à $4 \times 10^5$,

c) la composition de sucres suivante: $53 \pm 5\%$ de rhamnose, $21 \pm 2\%$ de glucose, $7,0 \pm 0,7\%$ de mannose, $6,0 \pm 0,6\%$ de galactose,

d) l'insolubilité dans les solvants organiques et la solubilité dans l'eau,

e) une réaction positive dans un test de développement de la couleur par la méthode au phénol-acide sulfurique,

f) un spectre d'absorption infrarouge, tel que représenté sur la figure 3,

g) un spectre d'absorption ultraviolet tel que représenté sur la figure 4.

2. Procédé pour la production d'une substance à base de polysaccharide selon la revendication 1, qui consiste à cultiver le microorganisme *Alteromonas vaga* MP-67, *FERM P-6281,* ou un de ses mutants capable de produire le polysaccharide selon la revendication 1, puis à récupérer le polysaccharide produit dans les milieux nutritifs.

10

3. Procédé selon la revendication 2, dans lequel le microorganisme est cultivé dans un milieu composé de sources de carbone, d'ions inorganiques et éventuellement de vitamines et d'acides aminés dissous dans de l'eau de mer, et même de l'eau de mer artificielle, dans des conditions aérées à une température de 10 à 35°C et le polysaccharide produit et accumulé dans le bouillon de fermentation est séparé et récupéré.

4. Procédé selon la revendication 3, dans lequel le microorganisme est cultivé à une température de 24 à 30°C.

5. Procédé selon la revendication 3, dans lequel la substance de polysaccharide est récupérée à partir du bouillon de fermentation en soumettant ce dernier à une séparation par centrifugation, en ajoutant un alcanol au liquide surnageant pour provoquer la précipitation au refroidissement et en isolant et purifiant le précipité par des procédés connus.

6. Microorganisme *Alteromonas vaga* MP-67, FERM P-6281.

7. Composition pharmaceutique contenant en tant qu'ingrédient actif une substance à base de polysaccharide selon la revendication 1, associée avec un véhicule pharmaceutiquement acceptable.

8. Substance à base de polysaccharide selon la revendication 1, utilisable comme médicament.

**Revendications pour l'Etat contractant: AT**

1. Procédé pour la production d'une substance à base de polysaccharide ayant:

a) un rapport carbone/azote de 20:1,

b) une masse moléculaire moyenne de $2 \times 10^5$ à $4 \times 10^5$,

c) la composition de sucres suivante: $53 \pm 5\%$ de rhamnose, $21 \pm 2\%$ de glucose, $7,0 \pm 0,7\%$ de mannose, $6,0 \pm 0,6\%$ de galactose,

d) l'insolubilité dans les solvants organiques et la solubilité dans l'eau,

e) une réaction positive dans un test de développement de la couleur par la méthode au phénol-acide sulfurique,

f) un spectre d'adorption infrarouge tel que reproduit sur la figure 3,

g) un spectre d'adsorption ultraviolet tel que reproduit sur la figure 4,

lequel procédé consiste à cultiver le microorganisme *Alteromonas vaga* MP-67, FERM P-6281, ou un de ses mutants capable de produire le polysaccharide et à récupérer le polysaccharide produit dans les milieux nutritifs.

2. Procédé selon la revendication 1, dans lequel le microorganisme est cultivé dans un milieu composé de sources de carbone, d'ions inorganiques et éventuellement de vitamines et d'acides aminés dissous dans de l'eau de mer, aussi bien que dans l'eau de mer artificielle, dans des conditions aérées à une température de 10 à 35°C et le polysaccharide produit et accumulé dans le bouillon de fermentation est séparé et récupéré.

3. Procédé selon la revendication 2, dans lequel le microorganisme est cultivé à une température de 24 à 30°C.

4. Procédé selon la revendication 2, dans lequel la substance de polysaccharide est récupérée à partir du bouillon de fermentation en soumettant ce dernier à une séparation par centrifugation, en ajoutant un alcanol au liquide surnageant pour provoquer la précipitation au refroidissement et en isolant et purifiant le précipité par des procédés connus.

0 084 333

FIG. 1

FIG. 2

FIG.3

FIG.4